Europäisches Patentamt

European Patent Office

Office européen des brevets

(19)

(11) Veröffentlichungsnummer : **0 399 479 B1**

(12) **EUROPÄISCHE PATENTSCHRIFT**

(45) Veröffentlichungstag der Patentschrift :
**11.08.93 Patentblatt 93/32**

(21) Anmeldenummer : **90109730.3**

(22) Anmeldetag : **22.05.90**

(51) Int. Cl.$^5$ : **A61K 9/68**

(30) Priorität : **26.05.89 CH 1989/89**

(43) Veröffentlichungstag der Anmeldung :
**28.11.90 Patentblatt 90/48**

(45) Bekanntmachung des Hinweises auf die
Patenterteilung :
**11.08.93 Patentblatt 93/32**

(84) Benannte Vertragsstaaten :
**AT CH DE ES FR IT LI**

(56) Entgegenhaltungen :
**WO-A-86/03967**
**AT-B- 350 728**
**DE-A- 2 922 670**
**FR-A- 2 320 083**
**GB-A- 2 181 646**
**US-A- 1 396 641**

(54) **Kaugummi zur Mund- und Rachendesinfektion, sowie Verfahren zu seiner Herstellung.**

(73) Patentinhaber : **Gergely, Gerhard, Dr.**
**Gartengasse 8**
**A-1050 Wien (AT)**

(72) Erfinder : **Gergely, Gerhard, Dr.**
**Gartengasse 8**
**A-1050 Wien (AT)**
Erfinder : **Tritthart, Wolfram, Dr.**
**Allgäu 36**
**A-9400 Wolfsberg (AT)**

(74) Vertreter : **Büchel, Kurt F., Dr.**
**Patentbüro Dr. Büchel Letzanaweg 25**
**FL-9495 Triesen (LI)**

**Beschreibung**

Die Erfindung betrifft einen Kaugummi nach dem Oberbegriff des Anspruches 1. Für die Desinfektion des Mund- und Rachenraumes bei grippalen Infekten und anderen, durch Viren, Bakterien bzw. Kokken verursachten Krankheitsbildern sind bisher im wesentlichen mit geeigneten Wirkstoffen beladene Gurgel- oder Sprühlösungen, sowie Lutschtabletten vorgeschlagen worden (Deutsche Apothekenzeitung 16.10.1986, Seite 2281ff). Beim Gurgeln erreichen die Wirkstoffe nur die vordere Mundhöhle und das nur kurzfristig; vom Verschlucken der Gurgellösung wird wegen der Konzentration abgeraten; Mandeln und Rachen werden so kaum behandelt. Besser sind daher Sprühlösungen, aber auch diese haben eine nur sehr kurze Einwirkungszeit. Lutschtabletten ermöglichen zwar eine Verlängerung der Einwirkungszeit, doch ist diese auch hier immer noch meist auf wenige Minuten beschränkt; ein vorzeitiges Zerbeissen und Verschlucken - insbesondere bei Kindern - ist nie ganz auszuschliessen. Hohe Konzentrationen von Cetylpyridiniumchlorid (CPC) wie auch von anderen Wirkstoffen können zu Schleimhautläsionen führen. Dies kann auftreten, wenn die Tabletten - wie bei Lutschtabletten üblich - direkt an den Schleimhäuten anliegen. Der mit dem Wirkstoff beladene Speichel kommt kaum in nennenswertem Umfang mit dem Plaquebelag auf den Zähnen in Kontakt, der ein bevorzugter Nährboden für Bakterien ist.

Alle diese Formulierungen finden auch Anwendung bei Entzündungen der Tonsillien, die einerseits Abwehrfunktionen gegen Infektionen haben, andererseits aber auch die Eintrittspforten für Mikroorganismen und Toxine in die Blutbahn sind. So sind pro ml Speichel $10^7$- $10^9$, pro Gramm Plaque $10^{11}$ - $10^{12}$ Mikroorganismen vorhanden. Meist ist die Veränderung der Abwehrlage durch virale Infektionen die Voraussetzung zum Ausbruch einer akuten Erkrankung der dann oft eine sekundäre bakterielle Infektion folgt. Als Antiseptika für die Mund- und Rachendesinfektion kommen Desinfizientien wie 8-Hydroxychinolin, 2,4-Dichlorbenzylalkohol, Kresole, Povidon-Jod, Hexetidin, Hexamidin, Chlorhexidin und quartäre Ammoniumverbindungen, im besonderen Cetylpyridiniumchlorid, Benzalkoniumchlorid, Dequaliniumchlorid in Frage. Ebenso werden Lokalantibiotika wie Bacitracin oder Tyrothricin zur Rachendesinfektion verwendet. Cetylpyridiniumchlorid hemmt in vitro bei einer Konzentration von 0,05 % innerhalb 30 Sekunden Einwirkdauer das Wachstum von Streptococcus pyrogenes, Streptococcus aureus und Candid albicans. $\alpha$-hämolysierende Streptokokken, sowie die oben genannten Streptococcus aureus und pyrogenes werden um 80 - 90 % auch in vivo reduziert. Cetylpyridiniumchlorid verhindert auch die Ausbildung von Zahnplaque. Eine Untersuchung mit artifizieller Zahnplaque aus Streptococcus mutans zeigte die Reduktion der Säurebildung durch Mikroorganismen bei Desinfektion mit 0,1%iger Cetylpyridiniumchlorid-Lösung. In einem vorläufigen Memorandum wird von der amerikanischen Gesundheitsbehörde FDA für Erwachsene und Kinder über 3 Jahren eine Dosierung von 0,025 - 0,1 % für Spüllösung und Lutschtabletten empfohlen. Bisher gibt es neben den desinfizierenden Sprays und Gurgellösungen zahlreiche Cetylpyridiniumchlorid-Tabletten in Dosierungen von 0,5 bis 2,5 mg pro Einzeldosis. So befindet sich in der US Pharmakopoea eine Monographie zu Cetylpyridiniumchlorid-Pastillen.

Um diese Nachteile zu vermeiden, hat man daher schon vorgeschlagen (FR-A-23 20083, 1977; US-A-1,396,641, 1921), das Desinfektionsmittel in einen Kaugummi einzubauen. Diesem (schon sehr alten!) Vorschlag stellten sich aber unerwartete Hindernisse in den Weg, so dass er sich bis heute nicht verwirklichen liess:

Bei konventioneller, in der Lebensmittelbranche üblicher Kaugummiherstellung wird der Wirkstoff in die Kaugummibase eingeknetet und damit vollkommen inkorporiert. Nun handelt es sich z.B. bei Cetylpyridiniumchlorid (CPC) um eine äusserst apolare, sehr gut fettlösliche Substanz. Besonders die langkettige Cetyl-Gruppe des Moleküls ist fettähnlich und auch gut emulgierbar. Wird nun CPC in Kaugummibase mit hohen Fettbestandteilen eingeknetet, ist die Freisetzung des wirksamen bestandteiles stark reduziert, beispielsweise auf maximal 20% innerhalb der Kauzeit von 15 bis 30 Minuten. Das französische Patent gibt sogar an, dass der Wirkstoff erst nach 15 Minuten freigesetzt wird. Damit ist aber die erwünschte Desinfektion des Mund- und Rachenraumes nicht möglich. Auch die AT-B-350 728 beschreibt ein Verfahren zur Herstellung eines medizinischen Kaugummis, bei dem ein in Methylenchlorid, gemeinsam mit 13,3 % Fetten oder Waschsen - gegebenenfalls auch mit Cellulosederivaten - gelöster, wasserlöslicher Wirkstoff mittels Vakuumtechnologie als Film auf die Kaugummibase aufgezogen und anschliessend verpresst wird, um die Wirkstoff-Freisetzung zu *verzögern* und damit eine Geschmacksverbesserung zu erzielen.

Die Erfindung hat sich daher die Aufgabe gestellt, einen Mund und Rachen desinfizierenden Kaugummi zu schaffen, der die besprochenen Nachteile herkömmlicher Zubereitungen nicht aufweist. Dies gelingt überraschend durch die im Kennzeichen des Anspruches 1 beschriebenen Merkmale. Vorteilhafte Weiterbildungen der Erfindung, sowie ein Verfahren zur Herstellung der erfindungsgemässen Kaugummi-Zubereitung sind in den Kennzeichen der Unteransprüche beschrieben.

Die Herstellung von Kaugummi aus Granulat ist zwar schon in der WO-A-8603967 beschrieben worden; doch wird dort ein Anteil an Fetten und/oder Wachsen von 20 - 40 Teilen gefordert. Grosse Anteile an Fetten oder Wachsen wirken sich aber aufgrund der erwähnten hohen Lipophilie des Wirkstoffes auf die Freisetzung negativ aus. Bei den erfindungsgemässen lipophilen Wirkstoffen wird daher schon mit 2 - 10 % Fettanteilen eine Verarbeitung der Granulatmischung zu Tabletten möglich, die auch später beim Kauen zur Ausbildung des Kaugummis führen.

Diese Reduktion des lipophilen Anteils führt überraschenderweise zu einer Steigerung der Wirkstofffreisetzung um mindestens 10 % auf bis zu etwa 45 % CPC innerhalb von 15 Minuten; dies ist aber zur Erlangung einer desinfizierenden Wirkkonzentration während der ersten Minuten der Kauzeit noch immer unbefriedigend. Wünschenswert wäre ein Anstieg der CPC-Freisetzung innerhalb von 10 Minuten auf 60 - 70 %.

Die an sich logische Erhöhung der löslichen Komponenten in der Tablettiermasse führte nicht zur gewünschten Erhöhung der Diffusion von CPC aus der Kaugummimatrix. Überraschenderweise kann durch Zusatz wenigstens einer der in Anspruch 3 genannten Substanzen die Wirkstofffreisetzung verbessert werden. Quervernetztes Polyvinylpyrrolidon wird üblicherweise bei der Herstellung von Tabletten als Sprengmittel zur Verbesserung der Zerfallszeit eingesetzt. Überraschenderweise führt jedoch ein Teil an quervernetztem Polyvinylpyrrolidon im erfindungsgemässen Kaugummi nicht nur zur üblichen verbesserten Sprengwirkung, sondern vor allem zu einer verbesserten Diffusion des Wirkstoffes CPC durch die und aus der Kaugummimatrix. Dies insbesondere dann, wenn - wie später noch erläutert - der Wirkstoff ubiquitär auf der Substanz aufgezogen wird. Dies verbessert die Freisetzung und ermöglicht eine ganz besonders kontinuierliche Wirkstoffabgabe während des Kauens. Diese Verbesserung der Diffusion aus einer Kaugummimatrix führt dazu, dass in der ersten Minute eine Initialdosis an CPC von etwa 25 - 30%, nach 3 Minuten von etwa 40 % und nach 10 Minuten von etwa 60% freigesetzt wird, d.h., dass der Anteil an freigesetztem CPC gegenüber der Grundformulierung um etwa 50 % erhöht ist. Nach einer Initialdosis von etwa 0,5 - 0,6 mg wird während eines Zeitraumes von 15 Minuten kontinuierlich CPC aus dem erfindungsgemässen Kaugummi freigesetzt; so entsteht eine desinfizierende Lösung mit entsprechender Wirkstoffkonzentration.

In der DE-A1 29 22670 war zwar schon ein Kaugummi vorgeschlagen worden, bei dem Wirkstoffe so eingebracht werden sollten, dass sie in porösen Teilchen, wie z.B. Aluminiumhydroxid, Aluminiumoxyd, zellulosehaltigen Gerüstbestandteilen, Calciumkarbonat o.dgl. eingelagert sind. Wie eingangs erwähnt, konnten sich die Kaugummis jedoch nicht durchsetzen, sei es aus herstellungstechnischen Schwierigkeiten, sei es weil die Wirkstoffabgabe doch nicht in befriedigender Weise sichergestellt werden konnte. Die erfindungsgemäss vorgeschlagenen Substanzen hingegen verbessern die Diffusion der Desinfektionsmittel aus dem Kaugummi in den Speichel.

Beim erfindungsgemässen Kaugummi liegt der Wirkstoff in dem therapeutischen System inkorporiert vor. Durch eine gleichmässige Wirkstofffreisetzung während etwa 10 Minuten werden die therapeutisch relevanten (und keine überhöhten!) Wirkstoffkonzentrationen mit Speichel gebildet, so dass das Risiko von Mikroläsionen reduziert ist.

Im besonderen ist vorgesehen, den erfindungsgemässen Kaugummi als Prophylaktikum gegen Karies und Gingivitis einzusetzen. Vom Cetylpyridiniumchlorid ist bekannt, dass Zahnplaque reduziert wird. Durch das Kauen der wirkstoffhältigen Kaugummimasse werden desinfizierende Wirkkonzentrationen direkt an die Zähne und das Zahnfleisch herangebracht, und es kommt gleichzeitig noch zu einem mechanischen Angriff auf den Plaque. Nur dadurch kann der gewünschte therapeutische Effekt erzielt werden. Daher ist auch die Reduktion des Zahnplaque hier günstiger als bei herkömmlichen Lutschtabletten.

Jedoch sind auch Dequaliniumchlorid (das allerdings einen etwas bitteren Geschmack aufweist) und Tyrotricin (das in der Vorbereitung besonderer Vorkehrungen bedarf), sowie Benzalkoniumchlorid, letzteres insbesondere in der Mischung mit CPC, im erfindungsgemässen Kaugummi mit Vorteil anzuwenden.

Die Herstellung der Wirkstoffmatrix erfolgt z.B. durch Aufziehen einer vorzugsweise alkoholischen Lösung des Wirkstoffes auf 20 - 60 % der vorgeschlagenen Substanz. Sie wird in einem Zwangsmischer vorgelegt, mit der alkoholischen Wirkstofflösung befeuchtet, homogen durchgemischt und anschliessend getrocknet.

Dieses Aufziehen des Wirkstoffes auf die Substanz ist zweckmässig, um den gewünschten Effekt der verbesserten Freisetzung zu erzielen und wirkt sich vorteilhaft auf seine gleichmässige Verteilung aus.

Beispiel 1: Tablettenkerne

| | I | II | III | IV | V |
|---|---|---|---|---|---|
| Gummibase | 100 | 100 | 100 | 100 | 100 |
| Fettsäuretriglycerid | 5 | 2 | 8 | 5 | 2 |
| Lactose | 90 | 56 | 100 | 80 | 56 |
| Sorbit | 26 | 63 | 13,2 | 36,35 | 63 |
| Quervernetztes PVP | 10 | 10 | 10 | 15 | 10 |
| Stärke | 5 | | 5 | | |
| Cetylpyridiniumchlorid | 0,4 | 0,4 | | | |
| Benzalkoniumchlorid | | | 0,1 | | |
| Dequaliniumchlorid | | | | 0,05 | |
| Tyrotricin | | | | | 0,4 |
| Talkum | 5 | 10 | 5 | 5 | 10 |
| Magnesiumstearat | 5 | 5 | 5 | 5 | 5 |
| Aroma | 12 | 12 | 12 | 12 | 12 |
| Süßstoff | 1,6 | 1,6 | | | |

| | VI | VII | VIII |
|---|---|---|---|
| Gummibase | 100 | 100 | 100 |
| Fettsäuretriglycerid | 5 | 5 | 5 |
| Lactose | 65,4 | 56 | 51 |
| Sorbit | 26 | 50 | 40 |
| Talkum | 10 | 8 | 10 |
| Magnesiumstearat | - | 4 | 5 |
| Aroma | 12 | 12 | 12 |
| Süßstoff | 1,6 | 3,0 | 1,6 |
| Wirkstoffmatrix | 40 | 22,0 | 35,5 |

EP 0 399 479 B1

Beispiel 2: Wirkstoffmatrix:

| | I | II | III | IV |
|---|---|---|---|---|
| Tyrotricin | 1,0 | | | |
| Cetylpyridiniumchlorid | | 1,0 | 2,0 | - |
| Benzalkoniumchlorid | | 1,0 | | |
| Dequaliniumchlorid | | | | 0,5 |
| Avicel® (mikrokristalline Zellulose | | 20 | | |
| Natriumalginat | 19 | | | |
| Na-carboxymethylcellulose | | | | 35 |
| Quervernetztes PVP | | 20 | 20 | |

Die Herstellung erfolgt ähnlich dem in WO-A-8603967 beschriebenen Verfahren.

100 Teile handelsübliche Kaugummibase, bestehend aus Latex und handelsüblichen Zuschlagstoffen, die in Tafeln geliefert werden, werden auf -10° C gekühlt, zerschlagen, mit einer gekühlten Mühle auf eine Korngrösse von 0,4 - 0,8 mm gemahlen und in dichtverschlossenen Polyäthylenbehältern bei 0° C aufbewahrt.

5 Teile Fettsäuretriglycerid werden am Wasserbad geschmolzen und auf eine vorgewärmte Mischung aus 90 Teilen Lactose und 26 Teilen Sorbit gebracht. Neben den in den obigen Beispielen angeführten Zuschlagstoffen Lactose und Sorbit sind auch andere Zuschlagstoffe wie Zucker, Glukose, Fruktose oder Mannitol, Maltodextrin und Dextrine möglich, obwohl man natürlich bevorzugt bei einem Cetylpyridiniumchlorid-Kaugummi Zuckerfreiheit anstreben wird. Es wird heiss gut durchgemischt und mit Kühlsole anschliessend auf 0° C kaltgerührt. In gekühltem Zustand wird die Masse auf eine Korngrösse von 0,2 - 0,5 mm gemahlen und bei 0° C aufbewahrt.

0,4 Teile Cetylpyridiniumchlorid werden in 4 Teilen Äthanol gelöst und in eine homogene Mischung aus 10 Teilen eines Sprengmittels, vorzugsweise z.B. quervernetztes PVP, und 5 Teilen Stärke homogen eingerührt. Als Sprengmittel sind neben den in Beispiel 2 genannten Verbindungen auch Pektine, Formaldehyd-Casein-Produkte und gehärtetes Rizinusöl möglich. Auch das Einarbeiten des Cetylpyridiniumchlorids in eine Fett- oder Ölphase ist denkbar. Anschliessend daran wird im Vakuum bei 60° C getrocknet. Die entstehende Pulvermischung wird durch ein Sieb der Maschenweite 0,2 mm gedrückt. Die Herstellung der weiteren Wirkstoffmatrix erfolgt analog den Zusammensetzungen gemäss Beispiel 2.

Man bringt die granulierte Gummibase und Fettbase in einen Vakuummischer ein, der mit Kühlsole auf 5° C gekühlt wird und fügt Festaromen, Süßstoffe und beispielsweise 15 Teile der Wirkstoffmatrix zu. Man evakuiert, um den Einfluss feuchter Luft und die Gefahr von deren Kondensation zu beseitigen, und mischt die Masse unter Heben und Schwenken des Vakuummischkessels durch. Sodann lässt man Luft einströmen, die maximal 10 % relative Feuchtigkeit enthalten darf und entlädt die fertige Mischung über ein rotierendes Sieb in Vorratsbehälter, die ebenfalls bei -5° bis 0° C gelagert werden.

Eine Variante zu dem oben beschriebenen Einbringen von CPC ergibt sich beim anschliessenden Dragieren der verpressten Kerne. Dabei wird CPC teilweise (der Restanteil an CPC wird dann in oben beschriebener Weise in die Matrix-Substanz eingebettet) oder auch vollständig in die Dragéedeckenmasse integriert. Durch wiederholtes, schichtweises Aufgiessen oder Aufsprühen der CPC-hältigen Suspensionslösung in einem zuerst kalten Dragéekessel bei anschliessender schrittweiser Erwärmung wird eine Dragéedecke von gewünschter Dicke erhalten. Die Dragierung kann auch zuckerfrei erfolgen, was natürlich für ein Produkt, das gegen die bildung von Zahnplaque eingesetzt werden kann, von Bedeutung ist.

Das Verpressen der Masse erfolgt vorzugsweise in gekühlten Tablettenmaschinen.

**Patentansprüche**

1. Kaugummi mit einem Gehalt an 0,05 bis 0,8 Gewichtsteilen eines - insbesondere apolaren bzw. schlecht wasserlöslichen - Mund- und/oder Rachendesinfiziens auf 100 Gewichtsteile Kaugummibase und/oder in einer diese umhüllenden Dragéeschicht, dadurch gekennzeichnet, dass die Kaugummibase in Granulatform und in Mischung mit 2 bis 10, vorzugsweise 5 bis 10 Gewichtsteilen wenigstens einer lipoiden Sub-

5

stanz vorliegt, vorzugsweise von Di- und Triglyceriden von Fettsäuren, höher viskosen Ölen, Wachsen oder Paraffinöl.

2. Kaugummi nach Anspruch 1, dadurch gekennzeichnet, dass als Desinfektionsmittel wenigstens eine der folgenden Verbindungen vorliegt: Cetylpyridiniumchlorid, Benzalkoniumchlorid, Dequaliniumchlorid, Tyrotricin.

3. Kaugummi nach Anspruch 1 oder 2, dadurch gekennzeichnet, dass er ausserdem 5 bis 20, vorzugsweise 10 bis 15 Gewichtsteile mindestens einer der folgenden Substanzen enthält: quervernetztes Polyvinylpyrrolidon, Formaldehyd-Casein-Produkte, gehärtetes Rhizinusöl.

4. Kaugummi nach einem der vorhergehenden Ansprüche, dadurch gekennzeichnet, dass er ausserdem zuckerfreie Zuschlagstoffe enthält.

5. Verfahren zur Herstellung eines Kaugummis nach einem der vorhergehenden Ansprüche, dadurch gekennzeichnet, dass 100 Gew.teile einer - vorzugsweise auf eine Korngrösse von 0,2 bis 1,0 mm - gemahlenen Kaugummibase mit 100 bis 150, vorzugsweise 115 bis 130 Gew.teilen von Pulverteilchen wenigstens eines Zuschlagstoffes, die mit 2 bis 10, vorzugsweise 5 bis 10 Gew.teilen Fett und/oder Wachs überzogen sind, unter Kühlung gemischt werden, wobei der Mischung und/oder einer Dragéeschicht auch 0.05 bis 0.8 Gewichtsteile eines Desinfektionsmittels zugesetzt und in gekühlten Vorrichtungen zu Tabletten verpresst werden.

6. Verfahren nach Anspruch 5, zur Herstellung einer Zubereitung nach Anspruch 3, dadurch gekennzeichnet, dass der Wirkstoff auf der Substanz aufgebracht wird, die anschliessend mit der granulierten Kaugummibase vermischt und zu Tabletten verpresst wird.

7. Verfahren nach Anspruch 5 oder 6, dadurch gekennzeichnet, dass die Verpressung in gekühlten Vorrichtungen erfolgt.

8. Verfahren nach einem der Ansprüche 5 bis 7, zur Herstellung von Tabletten mit einer Dragéeschicht, dadurch gekennzeichnet, dass die Tabletten mit der Dragéeschicht - vorzugsweise mehrschichtig - überzogen, dabei langsam auf eine Temperatur von 35 bis 60° C, z.B. während 10 bis 20 Min., erwärmt und anschliessend wieder abgekühlt werden.


**Claims**

1. Chewing gum containing 0.05 to 0.8 parts by weight of an oral and/or pharyngeal disinfectant - in particular a nonpolar or poorly water-soluble one - per 100 parts by weight of chewing gum base and/or in a tablet coat surrounding said base, characterised in that the chewing gum base is present in the form of granules and as a mixture with 2 to 10, preferably 5 to 10, parts by weight of at least one lipoid substance, preferably of di- and triglycerides of fatty acids, relatively highly viscous oil, waxes or liquid paraffin.

2. Chewing gum according to Claim 1, characterised in that at least one if the following compounds is present as a disinfectant: cetylpyridinium chloride, benzalkonium chloride, dequalinium chloride or tyrotricin.

3. Chewing gum according to Claim 1 or 2, characterised in that it furthermore contains 5 to 20, preferably 10 to 15, parts by weight of at least one of the following substances: crosslinked polyvinylpyrrolidone, formaldehyde/casein products or hydrogenated castor oil.

4. Chewing gum according to any of the preceding Claims, characterised in that it furthermore contains sugar-free additives.

5. Process for the preparation of a chewing gum according to any of the preceding Claims, characterised in that 100 parts by weight of a milled chewing gum base - preferably one milled to a particle size of 0.2 to 1.0 mm - are mixed, with cooling, with 100 to 150, preferably 115 to 130, parts by weight of powder particles of at least one additive which has been coated with 2 to 10, preferably 5 to 10, parts by weight of fat and/or wax, 0.05 to 0.8 part by weight of a disinfectant also being added to the mixture and/or a tablet coat and pressing to give tablets being effected in cooled apparatuses.

6. Process according to Claim 5, for the preparation of a formulation according to Claim 3, characterised in that the active ingredient is applied to the substance which is subsequently mixed with the granulated chewing gum base and pressed to give tablets.

7. Process according to Claim 5 or 6, characterised in that compression is effected in cooled apparatuses.

8. Process according to any of Claims 5 to 7 for the preparation of tablets having a tablet coat, characterised in that the tablets are coated with the tablet coat, preferably in several layers, slowly heated to a temperature of 35 to 60°C, for example in the course of 10 to 20 minutes, and then cooled again.

## Revendications

1. Gomme à mâcher contenant 0,05 à 0,8 partie en poids d'un désinfectant pour la bouche et le pharynx, notamment apolaire ou difficilement soluble dans l'eau, sur une base de 100 parties en poids de gomme à mâcher et/ou d'une couche dragéifiée enrobant cette base, caractérisée en ce que la base en gomme à mâcher se présente sous forme de granulés et est mélangée avec 2 à 10, de préférence 5 à 10 parties en poids d'au moins une substance lipoïde, de préférence de diglycérides et de triglycérides d'acides gras, d'huiles à viscosité élevée, de cires ou d'huile de paraffine.

2. Gomme à mâcher selon la revendication 1, caractérisée en ce qu'au moins l'une des combinaisons suivantes constitue l'agent désinfectant : chlorure de cétylpyridinium, chlorure de benzalkonium, chlorure de déqualinium, tyrothricine.

3. Gomme à mâcher selon la revendication 1 ou 2, caractérisée en ce qu'elle contient en outre 5 à 20, de préférence 10 à 15 parties en poids d'au moins une des substances suivantes: polyvinylpyrrolidone à réticulation transversale, produits de formaldéhyde-caséine, huile de ricin solidifiée.

4. Gomme à mâcher selon l'une des revendications précédentes, caractérisée en ce qu'elle contient en plus des additifs sans sucre.

5. Procédé pour fabriquer une gomme à mâcher selon l'une des revendications précédentes, caractérisé en ce que 100 parties en poids d'une base en gomme à mâcher moulue, dont la grosseur des grains est, de préférence, comprise entre 0,2 et 1,0 mm, sont mélangées sous refroidissement avec 100 à 150, de préférence 115 à 130 parties en poids de particules de poudre d'au moins un additif, ces particules étant enrobées de 2 à 10, de préférence de 5 à 10 parties en poids de graisse et/ou de cire, le mélange et/ou une couche dragéifiée étant additionnés de 0,05 à 0,8 partie en poids d'un agent désinfectant et comprimés dans des dispositifs refroidis pour former des comprimés.

6. Procédé selon la revendication 5 pour fabriquer une préparation selon la revendication 3, caractérisé en ce que l'agent actif est appliqué sur la substance qui est ensuite mélangée à la base granuleuse de gomme à mâcher et comprimée pour former des comprimés.

7. Procédé selon la revendication 5 ou 6, caractérisé en ce que la compression se fait dans des dispositifs refroidis.

8. Procédé selon l'une des revendications 5 à 7 pour fabriquer des comprimés dragéifiés, caractérisé en ce que les comprimés sont enrobés d'une, de préférence, de plusieurs couches dragéifiées, chauffés lentement à une température comprise entre 35 et 60° C, par exemple pendant 10 à 20 min., après quoi ils sont à nouveau refroidis.